# EUROPÄISCHE PATENTANMELDUNG

(11) **EP 1 250 917 A2**
(43) Veröffentlichungstag der Anmeldung: **23.10.2002**
(21) Anmeldenummer: 02007792.1
(22) Anmeldetag: 06.04.2002
(51) Int. Cl.: A61K 7/48, A61K 7/32, A61K 7/06, A61L 2/18, A61K 31/215, A23L 3/3454, B65B 55/00

(54) **Mittel zur Bekämpfung von Mikroorganismen, enthaltend primäre und sekundäre Ester des Polyglycerins in einem wirksamen Verhältnis**

(30) Priorität: 20.04.2001 DE 10119682
(71) Anmelder: Goldschmidt AG, 45127 Essen (DE)
(72) Erfinder: Brock, Achim, Dr., 45326 Essen (DE); Grüning, Burghard, Dr., 45134 Essen (DE); Hills, Geoffrey, Dr., 45355 Essen (DE)

(57) **Zusammenfassung**

Gegenstand der Erfindung sind Mittel zur Bekämpfung von Mikroorganismen, enthaltend primäre und sekundäre Ester des Polyglycerins in einem wirksamen Verhältnis von 8 : 1 bis 25 : 1.

## Beschreibung

Gegenstand der Erfindung sind Mittel zur Bekämpfung von Mikroorganismen, enthaltend primäre und sekundäre Ester des Polyglycerins in einem wirksamen Verhältnis.

Zur Bekämpfung von Mikroorganismen (grampositive Bakterien, gramnegative Bakterien, Mykobakterien, Dermatophyten, Sprossund Fadenpilze, Viren und Sporen) welche an der Oberfläche von Haut und Haaren, Kleidung, Geräten der Körperreinigung und -pflege wie beispielsweise im Dentalbereich, medizinischen Instrumenten aber auch Räumen und Einrichtungsgegenständen vorhanden sind, sind eine Vielzahl antimikrobiell wirksamer chemischer Substanzen bzw. Gemische dieser Substanzen bekannt, welche nach ihrem Verwendungszweck in Desinfektionsmittel, Konservierungsmittel, Antiseptika und kosmetische Wirkstoffe, um einige aufzuzählen, eingeteilt werden.

Die wesentlichen Vertreter dieser Gruppen sind: Aldehyde wie Formaldehyd, Glyoxal oder Glutaraldehyd; Phenol-Derivate wie 2.2'-Dihydroxybiphenyl und 4-Chlor-3-methylphenol; quaternäre Ammoniumverbindungen, Kationentenside wie Benzalkoniumchlorid, Cetrimoniumbromid, Cetylpyridiniumchlorid; Amphotenside sowie aktiven Sauerstoff abspaltende Verbindungen wie beispielsweise Wasserstoffperoxid, organische Persäuren, Alkylperoxide oder Alkylhydroperoxide.

Diese weisen allerdings etliche Nachteile auf, da sie die vielfältigen Forderungen, die in der Praxis an sie gestellt werden, wie z. B. breites Wirkungsspektrum, kurze Einwirkungszeiten bei niedrigen Temperaturen, gute Hautverträglichkeit, geringe Toxizität. Materialverträglichkeit, nicht oder nur unzureichend erfüllen.

Desinfektionsmittel auf Aldehyd- oder Phenolbasis gelten als toxikologisch und ökologisch bedenklich, führen häufig zu Sensibilisierungen insbesondere der Haut und Atmungsorgane und weisen darüber hinaus einen charakteristischen, durchdringenden und unangenehmen Geruch auf. Einige gelten zudem als potentielle Canzerogene.

Quaternäre Ammoniumverbindungen (Quats) sind toxikologisch weitestgehend unbedenklich, weisen keine oder nur sehr geringere Hautsensibilisierung auf und sind praktisch geruchlos. Sie besitzen jedoch eine beträchtliche hautreizende Wirkung. Bei Einsatz von Quats können sich wie bei der Verwendung von Aldehyden unerwünschte Ablagerungen und Schichten auf den behandelten Flächen bilden, die optisch nachteilig in Erscheinung treten und sich durch übliche Reinigungsverfahren nur schwer oder überhaupt nicht wieder entfernen lassen.

Aus der DE-A-42 37 081 sind kosmetische Deodorantien bekannt, welche als Wirkstoffe auf chemischem Wege hergestellte Fettsäureester des Di- und Triglycerins enthalten. Nach der dort gegebenen Lehre sind nur die primären Monoester des Diglycerins (Substitution in 1-Stellung) sowie die sekundären Monoester des Triglycerins (selektive Substitution in 2'-Stellung) wirksam zur Bekämpfung grampositiver Bakterien.

Diese regioisomerenreinen Monoester können gemäß den bekannten chemischen Verfahren des Standes der Technik (DE-A-38 18 293) hergestellt werden durch alkalisch katalysierte Umsetzung eines 1,5 bis 2,5-fachen molaren Überschusses an Fettsäuren oder Fettsäurederivaten mit Isopropylidenderivaten des Di- und Triglycerins, nachfolgender Reinigung des Reaktionsproduktes und anschließender saurer Hydrolyse oder Alkoholyse der Isopropylidengruppen. Nach Beendigung der Reaktion muss die Lösung neutralisiert und die Monoester müssen isoliert und gereinigt werden. Neben der Vielstufigkeit der Synthese aufgrund der Notwendigkeit von Schutzgruppenchemie ist im Falle der Diglycerin-Derivate zusätzlich die Verwendung von equimolaren Mengen an Epichlorhydrin als Nachteil dieser Route zu bewerten.

Daneben sind auch enzymatisch katalysierte Verfahren zur Herstellung von primär substituierten Polyglycerinfettsäureestern bekannt. D. Charlemagne und M. D. Legoy (JAOCS 1995, Vol. 72, no. 1, 61-65) adsorbieren hierzu erst das Polyglycerin auf der gleichen Menge Kieselgel, bevor sie in Suspension mit Fettsäuremethylestern unter Lipasekatalyse reagieren lassen. Nachteilig ist hierbei vor allem der Verlust des teuren Enzyms, das nach Beendigung der Reaktion zusammen mit dem Kieselgel durch Filtration abgetrennt wird. Zudem berichten sie in Übereinstimmung mit dem Stand der Technik (R. Lortie, M. Trani, F. Ergan, Biotechnol. Bioeng. 1993, 41, 1021), dass bei Verwendung von 1,3-spezifischen Lipasen Isomerisierungen durch Acylmigration von primären zu sekundären Positionen beobachtet werden. S. Matsumura, M. Maki, K. Toshima und K. Kawada (J. Jpn. Oil Chem. Soc. 1999, Vol. 48, No. 7, 681-692) nutzen dieses Verfahren in Abwandlung, um unter Einsatz von 20 Gew.-% Enzym Polyglycerinester zu synthetisieren. Gemäß der in der DE-A-42 37 081 vermittelten Lehre reinigen sie unter hohem Aufwand mittels Säulenchromatographie weiter auf, um zu reinen Monoestern mit den bekannten antimikrobiellen Aktivitäten zu gelangen.

Zusammengefasst besagen also die Lehren des Standes der Technik, dass nur die primären Diglycerinmonoester sowie die sekundären Triglycerinmonoester geeignete Mittel zur Bekämpfung von Mikroorganismen sind. Diese Verbindungen sind unter hohem präparativen Aufwand erhältlich. Mischungen von Regioisomeren der reinen Monoester sind bekannt. Sie besitzen vergleichbare antimikrobielle Eigenschaften. Ihre Darstellung ist jedoch ebenfalls mit einem hohen Synthese- und Reinigungsaufwand verbunden.

Aufgabe der Erfindung war es daher, Mittel zur Bekämpfung von Mikroorganismen zu finden, die den geschilderten Nachteilen der Mittel des Standes der Technik weitgehend Abhilfe schaffen, eine hohe antimikrobielle Wirkung zeigen und aus einfach zugänglichen Rohstoffen nach einem ökonomisch tragbaren und ökologisch unbedenklichen Verfahren unkompliziert hergestellt werden können.

Es war überraschend und für den Fachmann aufgrund der Lehren des Standes der Technik nicht vorauszusehen, dass auch Mischungen von Fettsäuremono-, -di- und -triestern des Polyglycerins, die jeweils primäre und sekundäre Mono-, Di- und Triester des Polyglycerins in einem wirksamen Verhältnis enthalten, vergleichbare und teilweise sogar deutlich bessere Wirksamkeiten bei der Bekämpfung von Mikroorganismen aufweisen, als die durch chemische Synthese oder enzymatische Herstellung und Aufreinigung hergestellten Monoester.

Ein Gegenstand der Erfindung sind daher Mittel zur Bekämpfung von Mikroorganismen, die dadurch gekennzeichnet sind, dass sie einen Gehalt an Mischungen von Fettsäureestern des Polyglycerins enthaltend primäre und sekundäre Ester des Polyglycerins in einem wirksamen Mol-Verhältnis aufweisen und einfach in einem Schritt aus den Rohstoffen Polyglycerin und Fettsäure bzw. Fettsäurederivat herzustellen sind.

Ein weiterer Gegenstand der Erfindung ist die Verwendung von antimikrobiell wirksamen Mischungen von Fettsäuremonoestern und Fettsäurediestern des Polyglycerins, die einen Gehalt an primären und sekundären Estern des Polyglycerins in einem wirksamen Verhältnis aufweisen, insbesondere des Di- und/oder Triglycerins zur Herstellung von Desinfektionsmitteln, Sterilisationsmitteln, Antiseptika, Konservierungsmitteln, die sich zur Sterilisation und Desinfektion von Flächen und chirurgischen Instrumenten sowie zur Konservierung, insbesondere zur Konservierung von kosmetischen oder dermatologischen Zubereitungen, eignen.

Die Mittel eignen sich darüber hinaus auch zur Konservierung von Lebensmitteln und können auch zur antimikrobiellen Ausrüstung von Lebensmittelverpackungen eingesetzt werden.

Die erfindungsgemäßen antimikrobiellen Mittel eignen sich insbesondere, auch aufgrund ihrer Mildheit, zur Herstellung kosmetischer Präparate zur Bekämpfung des Körpergeruches, zur Bekämpfung von Schuppen und zur Bekämpfung unreiner Haut.

Weitere Gegenstände der Erfindung sind durch die Ansprüche gekennzeichnet.

Die erfindungsgemäß verwendeten Polyglycerine sind zum einen lineare Verbindungen der allgemeinen Formel

HO-CH₂-CH(OH)-CH₂-O-[CH₂-CH(OH)-CH₂-O]ₙ-H

worin n = 1-9 vorzugsweise 1-6, insbesondere 1-3, speziell 1 und 2 bedeutet. Darüber hinaus können die verwendeten Polyglycerine auch verzweigt sein und cyclische Anteile enthalten.

Es sind bei Raumtemperatur hoch viskose Flüssigkeiten, welche neben Diglycerin vor allem die höher kondensierten Oligomeren des Glycerins enthalten. Besonders bevorzugt im Sinne der vorliegenden Erfindung werden technische Gemische von Polyglycerinen eingesetzt, die üblicherweise Diglycerin, Triglycerin, Tetraglycerin und Pentaglycerin enthalten.

Sie können beispielsweise industriell hergestellt werden durch basenkatalytische Kondensation von Glycerin oder auch durch Hydrolyse und Kondensation von Epichlorhydrin. Darüber hinaus sind Polyglycerine auch zugänglich durch Polymerisation von Glycidol. Die Trennung und Isolation der einzelnen Polyglycerine ist durch Behandlung mit den verschiedenen im Stand der Technik bekannten Mitteln möglich. Eine Übersicht von G. Jakobson über die verschiedenen Syntheserouten ist zu finden in "Fette Seifen Anstrichmittel", 1986, 88. Jahrgang, Nr. 3, 101-106. Die verschiedenen Strukturmöglichkeiten für Polyglycerin können nachgelesen werden bei H. Dolhaine, W. Preuß und K. Wollmann (Fette Seifen Anstrichmittel 1984, 86. Jahrgang, Nr. 9, 339-343).

Handelsübliche Produkte sind im Allgemeinen Gemische von Polyglycerinen mit verschiedenen Kondensationsgraden, der maximale Kondensationsgrad kann darin in der Regel bis zu 10, in Ausnahmen auch größer sein. Sie enthalten etwa 0 bis 5 Gew-% Glycerin, 15 bis 40 Gew-% Diglycerin, 30 bis 55 Gew-% Triglycerin, 10 bis 25 Gew-% Tetraglycerin, 0 bis 10 Gew-% höhere Oligomere.

Die erfindungsgemäß bevorzugt verwendeten Polyglycerine enthalten 15 bis 35 Gew-% Diglycerin, 38 bis 52 Gew-% Triglycerin, 15 bis 25 Gew-% Tetraglycerin, < 10 Gew-% höhere Oligomere und < 2 Gew-% cyclische Verbindungen. Besonders bevorzugt werden Polyglycerine, die nur oder überwiegend Diglycerin enthalten, verwendet.
Die im Sinne der vorliegenden Erfindung vorzugsweise einzusetzenden Fettsäuren und Fettsäurederivate sowie deren Gemische leiten sich von gradkettigen oder verzweigten, gesättigten, einfach oder mehrfach ungesättigten Carbon- und Fettsäureresten mit 6 bis 14 C-Atomen, vorzugsweise 8 bis 12, insbesondere 8 bis 10 C-Atomen in der Hauptkette ab.

Als Fettsäurederivate können alle üblichen Derivate eingesetzt werden, die Ver-/Umesterungsreaktionen eingehen. Erfindungsgemäß sind die Fettsäurederivate besonders bevorzugt ausgewählt aus Fettsäurealkylestern mit 1 bis 4 C-Atomen im Alkoholrest.

Als Fettsäuren oder deren Ester werden einzeln oder in Mischungen Fettsäuren wie Capronsäure, Caprylsäure, Caprinsäure, 2-Ethylhexansäure, Undecylensäure, Laurinsäure und Myristinsäure verwendet. Geeignet sind prinzipiell alle Fettsäuren mit ähnlicher Kettenverteilung.

Vorzugsweise werden Caprylsäure und Caprinsäure verwendet.

Das Stoffmengenverhältnis von Fettsäure oder Fettsäurederivaten zu Polyglycerin wird so eingestellt, dass im Reaktionsgemisch ein Überschuss an Hydroxylgruppen gegenüber Fettsäureresten besteht. Bevorzugt im Sinne der vorliegenden Erfindung ist es, das Stoffmengenverhältnis von Mol Fettsäurederivaten zu Mol Polyglycerin im Verhältnis von 0,25:1 bis 4:1, insbesondere 0,5:1 bis 2:1 einzustellen.

Die Ver- und Umesterungsreaktion zu den antimikrobiell wirksamen Mischungen von Fettsäuremonoestern und Fettsäurediestern des Polyglycerins, die einen Gehalt an primären und sekundären Estern des Polyglycerins in einem wirksamen Verhältnis aufweisen, kann mittels Enzymen, insbesondere immobilisierter Enzyme, vorzugsweise mit solchen Enzymen ausgeführt werden, die ausgewählt sind aus der Gruppe der Lipasen, Esterasen oder Proteasen, insbesondere Lipasen. Sie besitzen Enzymkatalyseaktivität für Esterbindungen, insbesondere zur Hydrolyse, Ver- und Umesterung. Derartige Lipasen sind in der WO 90/09451 beschrieben. Darüber hinaus ist das Produkt Novozym® 435 der Firma Novozymes als immobilisiertes Lipasesystem bekannt und im Handel erhältlich. Dieses Enzym wird besonders bevorzugt im Sinne der vorliegenden Erfindung eingesetzt.

Eine gaschromatographische und eine kernresonanzspektroskopische Methode zur Analyse und Charakterisierung von partiell veresterten Polyglycerinen wird von R. Gerhards beschrieben (Vortrag "Trends in der Analytik von kosmetischen Rohstoffen" im Rahmen des 14. DGK-Symposiums "Innovative Analytik in der Kosmetik - Anforderungen, Applikationen, Trends", 04.-06.2001, Congress Centrum Hamburg). Die Kombination der dort dargestellten Methoden ist geeignet zur Identifizierung und Quantifizierung der primären und sekundären Monoester des Di- und Polyglycerins und ist somit insbesondere geeignet zur Charakterisierung des wirksamen Verhältnisses von primären und sekundären Isomeren der erfindungsgemäßen Polyglycerinester. Besonders im Fall von Diglycerinmonoestern liefert die Gaschromatographie eine präzise quantitative Aussage über das Verhältnis von isomeren primären zu sekundären Estern des linear aufgebauten Diglycerins. Hierbei können die Diglycerinmonoester in reiner Form, in Mischungen von Diglycerinestern unterschiedlichen Veresterungsgrades wie auch als Bestandteil einer Mischung von verschiedenen Polyglycerinen und verschiedenen Veresterungsgraden vorliegen. Durch Bilden des Quotienten der Flächenprozente von definierten Peaks im Retentionsbereich des Diglycerinmonoesters ergibt sich das molare Verhältnis von primären zu sekundären Monoesterisomeren des linear aufgebauten Diglycerins.

Die erfindungsgemäßen Polyglycerinfettsäureester bestehen zusammenfassend aus einem Gemisch von Verbindungen unterschiedlichen Veresterungsgrades, wobei sich jeder Veresterungsgrad aus einem Gemisch von primär und sekundär substituierten Isomeren in einem wirksamen Verhältnis zusammensetzt. Beträchtliche Anteile nicht veresterten Polyglycerins können enthalten sein. Das zu Grunde liegende Polyglycerin kann dabei einheitlich oder seinerseits wiederum ein Gemisch von Produkten unterschiedlichen Kondensationsgrades sein.

Die erfindungsgemäßen Mittel zur Bekämpfung von Mikroorganismen können je nach Einsatzzweck darüber hinaus noch auf diesem Gebiet übliche anionische, nichtionische, kationische und/oder amphotere Tenside enthalten.

Typische Beispiele für solche Tenside sind:
1. Nichtionische Tenside basierend auf Alkylenoxiden wie Ethoxylate langkettiger verzweigter Alkohole, Ethoxylate von Sorbitanestern, Propylenoxidethylenoxidcopolymere, Hydroxyalkylfettsäureamide, Polydimethylsiloxanpolyalkylenoxidcopolymere, Zucker basierte Tenside wie Alkylpolyglycoside, Alkylglycosidester, N-Acylglucamide und Polyglycerinester,
2. Anionische Tenside wie Alkyl- und Alkylethersulfate, α-Olefinsulfonate, Fettsäureestersulfonate, Alkylarylsulfonate, Sulfosuccinate, Phosphorsäurealkyl- oder alkoxyalkylester, Taurate, N-Acylaminosäurederivate, Sarcosinate, Isethionate und Seifen,
3. Kationische Tenside wie Alkyltrimethylammoniumsalze, Fettsäureester von Di- oder Triethanolammoniumsalzen, Alkylimidazoliniumsalzen, Acylamidopropyldimethylammoniumsalze, kationisch derivatisierte Polydimethylsiloxane,
4. Zwitterionische und amphotere Tenside wie Betaine, Sulfobetaine, Aminoxide und Amphoacetate.

Bei den erfindungsgemäßen Mitteln zur Bekämpfung von Mikroorganismen handelt es sich beispielsweise um Sterilisationsmittel, Desinfektionsmittel, desinfizierende Reinigungsmittel, Allzweckreiniger, Sanitärreiniger, Badreiniger, Maschinengeschirrspülmittel, Waschmittel, kosmetische Reinigungs- und Pflegemittel. Kosmetische Mittel auf Basis der beschriebenen Polyglycerinfettsäureester werden insbesondere eingesetzt zur Bekämpfung von Körpergeruch, von Schuppen oder zur Bekämpfung von Unreinheiten der Haut. Sie können als solche in Form homogener Flüssigkeiten, als Gele, als Salben, als wachsartige oder emulsionsartige Zubereitungen formuliert sein. Insbesondere in der Emulsionsform werden sie Öle wie Esteröle, flüchtige oder weniger flüchtige Siliconderivate wie Decamethylcyclopentasiloxan, Paraffinöle und dergleichen enthalten.

Es kann von Vorteil sein, in den erfindungsgemäßen Mitteln zur Bekämpfung von Mikroorganismen andere antimikrobiell wirksame Stoffe mitzuverwenden. Als solche seien genannt Triclosan, Farnesol, Glycerinmonolaurat oder 2-Ethylhexyloxyglycerin. Sie können je nach Anwendungszweck neben den genannten Tensiden noch die jeweils spezifischen Hilfsund Zusatzstoffe, beispielsweise Lösungsmittel, Gerüststoffe, Schauminhibitoren, Salze, Bleichmittel, Bleichaktivatoren, optische Aufheller, Vergrauungsinhibitoren, Lösungsvermittler, Verdickungsmittel, Duft- und Farbstoffe, Emulgatoren, biogene Wirkstoffe wie Pflanzenextrakte und Vitaminkomplexe enthalten. Als Lösungsmittel kommen insbesondere in Frage Wasser oder Alkohole wie beispielsweise Ethanol, Propanol, Isopropanol, 2-Methyl-2-propanol, Propylenglykol, Dipropylenglykol oder Glycerin.

Die jeweils einzusetzenden Mengen an derartigen Zusatzstoffen sind in Abhängigkeit von der Art des jeweiligen Produktes dem Fachmann bekannt oder können im Bedarfsfall durch einfaches Ausprobieren leicht ermittelt werden.

Weitere Verwendungsmöglichkeiten für die erfindungsgemäßen Mittel ist deren Einsatz als Konservierungsmittel in Lebensmitteln und in Lebensmittelverpackungen, in denen sie in der Regel in Konzentrationen von 0,01 bis 5 Gew-%, bevorzugt 0,1 bis 1 Gew-% eingesetzt werden. Lebensmitteln können die erfindungsgemäßen Ester einfach in entsprechender Menge zugesetzt werden. Zur Anwendung in Verpackungen kommen die Polyglycerinester, indem beispielsweise Papiere mit einer Lösung oder Emulsion der Ester getränkt werden oder Folien mit entsprechenden Zubereitungen der Ester besprüht werden. Die Ester können auch vor oder während des Formgebungsprozesses der Verpackungen wie der Extrusion zugesetzt werden.

Die nachfolgenden Ausführungsbeispiele stellen bevorzugte Umsetzungen der vorliegenden Erfindung dar, sind jedoch nicht geeignet, die Erfindung hierauf zu beschränken.

### Beispiel 1: Diglycerincaprinat

In einem Dreihalskolben mit KPG-Rührer und aufgesetzter Destillationsbrücke werden 415 g Diglycerin (erhältlich von Solvay Alkali GmbH) und 431 g Caprinsäure eingewogen und bei 60 °C mit 16,9 g Novozym® 435 versetzt. Das entstehende Reaktionswasser wird im Wasserstrahlvakuum entfernt, bis die Säurezahl der Reaktionsmischung auf einen Wert kleiner 2 gefallen ist. Zur Abtrennung des Enzyms wird das Produkt abschließend filtriert.

### Beispiel 2: Polyglycerin-3-caprinat

In einem Dreihalskolben mit KPG-Rührer und aufgesetzter Destillationsbrücke werden 460 g eines Polyglycerins gekennzeichnet durch folgende Verteilung (Gew-%): 0,2 Glycerin, 32,6 Diglycerin, 41,2 Triglycerin, 14,8 Tetraglycerin, 3,9 Pentaglycerin, 1,9 Hexaglycerin, 5,4 höhere Polyglycerine und 345 g Caprinsäure eingewogen und bei 60 °C mit 16,1 g Novozym® 435 versetzt. Das entstehende Reaktionswasser wird im Wasserstrahlvakuum entfernt, bis die Säurezahl der Reaktionsmischung auf einen Wert kleiner 2 gefallen ist. Zur Abtrennung des Enzyms wird das Produkt abschließend filtriert.

### Beispiel 3: Diglycerincaprylat

In einem Dreihalskolben mit KPG-Rührer und aufgesetzter Destillationsbrücke werden 415 g Diglycerin (erhältlich von Solvay Alkali GmbH) und 361 g Caprylsäure eingewogen und bei 60 °C mit 15,5 g Novozym® 435 versetzt. Das entstehende Reaktionswasser wird im Wasserstrahlvakuum entfernt, bis die Säurezahl der Reaktionsmischung auf einen Wert kleiner 2 gefallen ist. Zur Abtrennung des Enzyms wird das Produkt abschließend filtriert.

### Beispiel 4: Polyglycerin-3-caprylat

In einem Dreihalskolben mit KPG-Rührer und aufgesetzter Destillationsbrücke werden 579 g eines Polyglycerins gekennzeichnet durch folgende Verteilung (Gew-%): 0,2 Glycerin, 32,6 Diglycerin, 41,2 Triglycerin, 14,8 Tetraglycerin, 3,9 Pentaglycerin, 1,9 Hexaglycerin, 5,4 höhere Polyglycerine und 363 g Caprylsäure eingewogen und bei 60 °C mit 18,8 g Novozym® 435 versetzt. Das entstehende Reaktionswasser wird im Wasserstrahlvakuum entfernt, bis die Säurezahl der Reaktionsmischung auf einen Wert kleiner 2 gefallen ist. Zur Abtrennung des Enzyms wird das Produkt abschließend filtriert.

### Beispiel 5: Triglycerinlaurat

In einem Dreihalskolben mit KPG-Rührer und aufgesetzter Destillationsbrücke werden 480 g Triglycerin (erhältlich von Solvay Alkali GmbH) und 401 g Laurinsäure eingewogen und bei 60 °C mit 17,6 g Novozym® 435 versetzt. Das entstehende Reaktionswasser wird im Wasserstrahlvakuum entfernt, bis die Säurezahl der Reaktionsmischung auf einen Wert kleiner 2 gefallen ist. Zur Abtrennung des Enzyms wird das Produkt abschließend filtriert.

### Beispiel 6: Bestimmung des Verhältnisses von primären zu sekundären Estern

Das Verhältnis von primären zu sekundären Monoestern wird gemäß der angeführten Methode nach dem Stand der Technik bestimmt.

### Folgender Versuchsaufbau liegt zu Grunde:

Säule: BPX 70 (der Fa. SGE); 50 m x 0,22 mm Innendurchmesser x 0,25 µm Filmdicke Retention Gap: 5 m x 0,32 mm Innendurchmesser Gaschromatograph: HP 6890 (FID) Starttemperatur: 80 °C Aufheizrate: 3 °C/min. Endtemperatur: 260 °C
Probenvorbereitung: Nach der Silylierung der Probe (50 mg Einwaage in 1 ml Pyridin) mit 0,5 ml MSTFA über eine Stunde bei Raumtemperatur wird der Rest des vorhandenen MSTFA durch langsame Zugabe von 50 µl Methanol (bis zu 10 mal) abreagiert.

Wie beschrieben werden die Peaks im Retentionsbereich des Diglycerinmonoesters ausgewertet. Hierzu bestimmt man den Quotienten "q" der Flächenprozente folgender Peaks. Im Fall der Caprinsäurederivate werden die Flächenprozente der Peaks bei 39,35 und 39,45 min. (lineares Diglycerin in 1-Stellung substituiert) addiert und durch die Flächenprozente des Peaks bei 38,95 min. (lineares Diglycerin in 2-Stellung substituiert) dividiert. Ein entsprechendes Muster von Peaks ergeben auch diejenigen Diglycerinmonoester, die andere Säurereste als Caprinreste tragen. Hierbei kommt es zu gleichen Verschiebungen der Retentionszeiten für die Peaks jedes einzelnen Isomers, weitere Verschiebungen treten in Abhängigkeit von Messparametern wie z. B. der Flussrate auf. Dies verändert jedoch weder die relative Lage noch die relativen Intensitäten der Peaks der aufgeführten relevanten Isomere zueinander. Der resultierende Wert des Quotienten "q" gibt daher immer das molare Verhältnis von primären zu sekundären Estern des linearen Diglycerins in den erfindungsgemäßen Produkten an. Beispielhaft wird die Ermittlung dieses Wertes an 4 Produkten aufgezeigt.

Zur Auswertung wird die Fläche des Kopfpeaks A als Divisor, die Summe der Flächen der beiden Hinterpeaks B+C als Divident herangezogen. Der Quotient q gibt dann direkt das molare Verhältnis von primären zu sekundären Monoestern wieder. 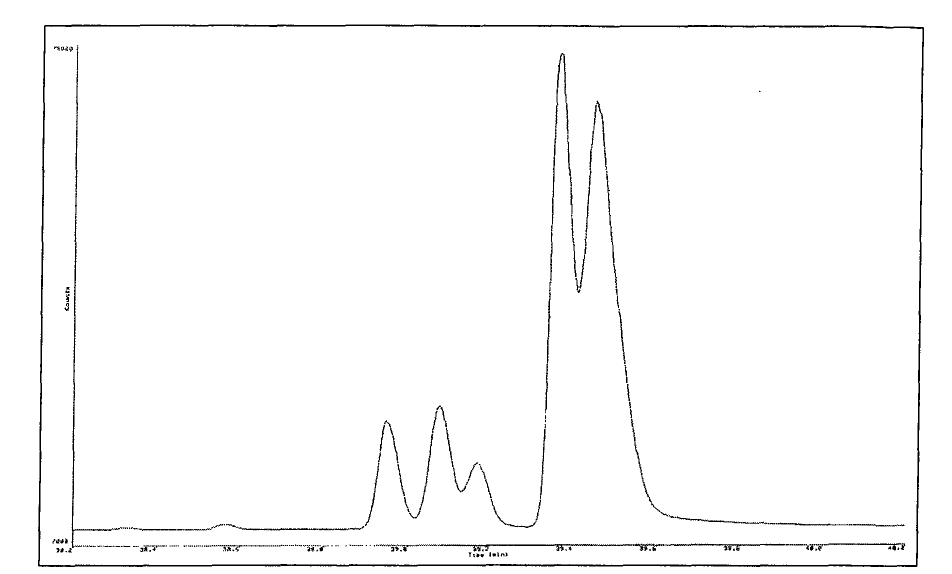 Diglycerincaprinat (Ausführungsbeispiel 1); q = 14,2 = (B+C)/A 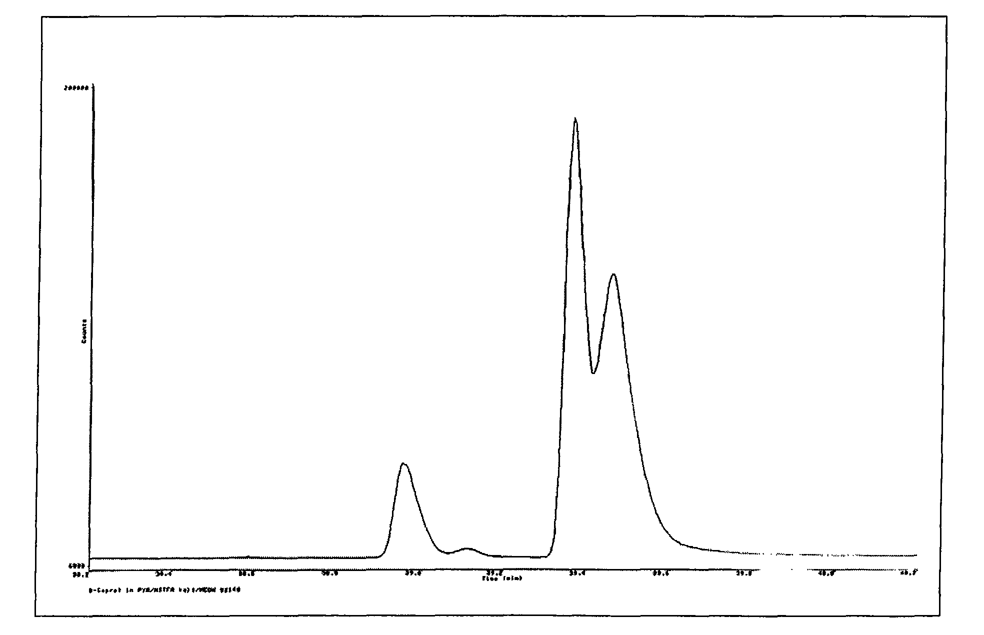 D-Caprate A (Solvay Alkali GmbH); q = 6,9 = (B+C)/A 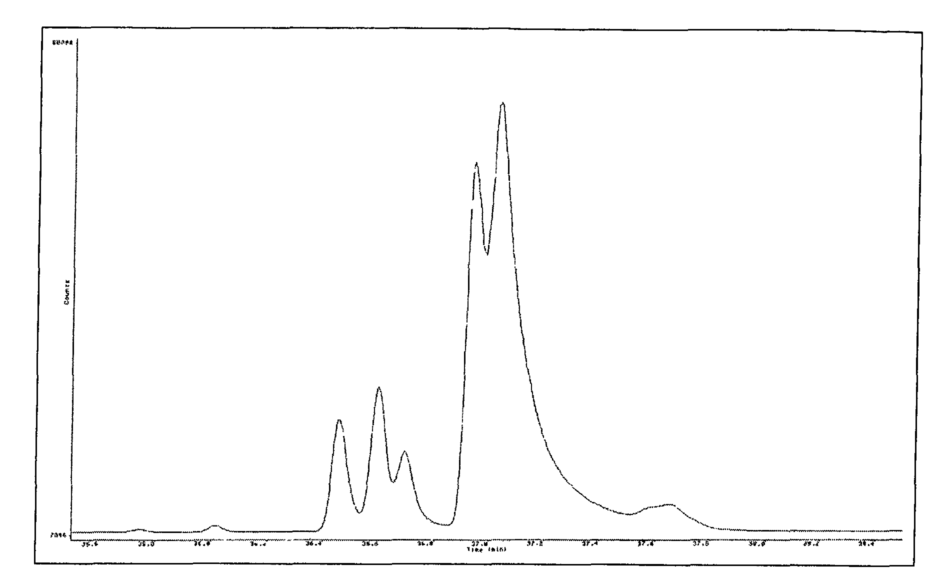 Diglycerincaprylat (Ausführungsbeispiel 3); q = 16,1 = (B+C)/A 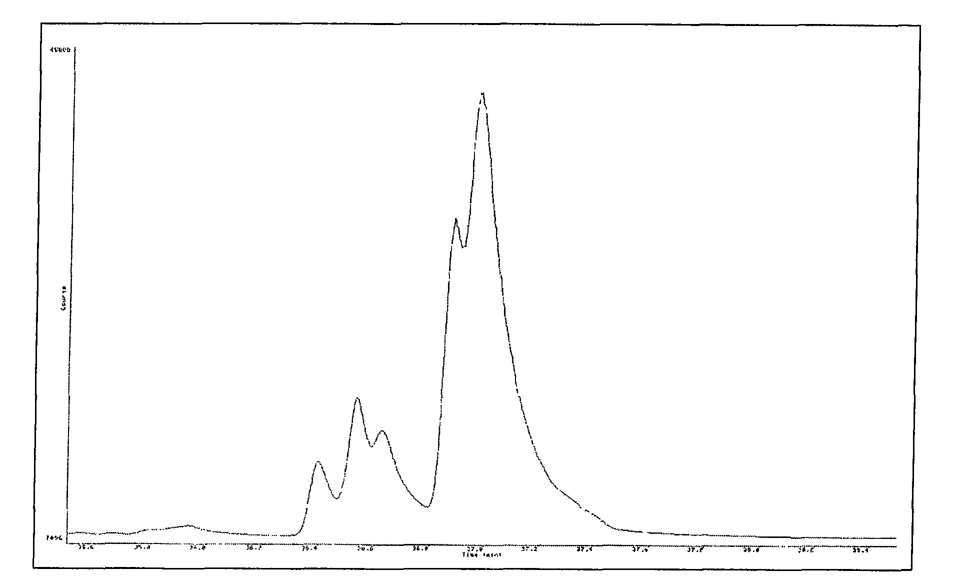 Polyglycerin-3-caprylat (Ausführungsbeispiel 4); q = 17,7 = (B+C)/A

Nach der Lehre gemäß dem Stand der Technik sind nur die primären Monoester des Diglycerins (Substitution in 1-Stellung) sowie die sekundären Monoester des Triglycerins (selektive Substitution in 2'-Stellung) wirksam zur Bekämpfung grampositiver Bakterien. Die Bestimmung der antimikrobiellen Aktivität gegenüber Mikroorganismen von erfindungsgemäßen Produkten zeigt hingegen, dass auch Mischungen von primären und sekundären Polyglycerinestern in einem wirksamen Verhältnis zumindest vergleichbare, in der Regel sogar deutlich überlegene Wirksamkeiten besitzen. Bevorzugt im Sinne einer sehr effizienten antimikrobiellen Wirksamkeit sind hierbei Mischungen von primären und sekundären Polyglycerinestern, die einen Wert "q" zwischen 8 bis 25, besonders bevorzugt zwischen 10 bis 20 aufweisen. Gemische mit Werten für "q" außerhalb dieses Bereiches, d. h. die reinen primären Monoester des Diglycerins (q→∞) sowie die reinen sekundären Monoester des Triglycerins (q→0) oder Mischungen mit einem unwirksamen Verhältnis (q>25; q<8) besitzen unterlegene Wirksamkeiten. Festgestellt wird die Wirksamkeit der erfindungsgemäßen Produkte mit Hilfe der "Prüfung auf ausreichende Konservierung" (gemäß der Europäischen Pharmaverordnung).

### Beispiel 7: Durchführung der mikrobiologischen Tests:

### A) Gegen Corynebacterium xerosis, Staphylococcus epidermidis und Candida albicans

### 1. Proben und Material

1.1. Proben
a. Diglycerinmonocaprinat (D-Caprate A, Solvay Alkali GmbH; Vergleichssubstanz gemäß dem Stand der Technik)
b. Diglycerincaprinat (Ausführungsbeispiel 1)
c. Polyglycerin-3-caprinat (Ausführungsbeispiel 2)
d. Diglycerincaprylat (Ausführungsbeispiel 3)
e. Polyglycerin-3-caprylat (Ausführungsbeispiel 4)
f. Triglycerinmonolaurat (T-Laurate A, Solvay Alkali GmbH; Vergleichssubstanz gemäß dem Stand der Technik)
g. Triglycerinlaurat (Ausführungsbeispiel 5)

1.2. Testkeime
Corynebacterium xerosis DSM 20743
Staphylococcus epidermidis DSM 3269
Candida albicans ATCC 10231

1.3. Verwendete Medien Nährmedien
CSL: Caseinpepton-Sojamehlpepton-Lösung
CSA: Caseinpepton-Sojamehlpepton-Agar
Sabouraud-Glucose-Bouillon/Agar
Verdünnungsflüssigkeit mit Inaktivierungszusätzen
NaCl-Pepton-Pufferlösung mit EG-Enthemmer (3 % Tween® 80,
0,3 % Lecithin, 0,1 % Histidin, 0,5 % Na-Thiosulfat)

### 2. Methode

2.1. Herstellen der Testlösungen
Von jeder Probe wurden am Vortag der Untersuchung Testlösungen von 0,1 % (w/v) (Proben a-f) bzw. von 0,5 % (w/v) (Proben g, h) in CSL angesetzt. Dazu wurden je 100 ml CSL im Wasserbad auf 60 °C temperiert. Von jeder Probe wurde 0,1 g (Proben a-f) bzw. 0,5 g (Proben g, h) in jeweils 100 ml CSL von 60 °C eingewogen. Die Ansätze wurden von Hand kräftig geschüttelt und bei 30 °C über Nacht im Brutschrank belassen.

2.2. Herstellen der Testkeimsuspensionen
Corynebacterium xerosis über 3 bis 4 Tage anzüchten. Übrige Keime in Bouillon bzw. durch Abschwemmen gewinnen.

2.3. Kontamination der Proben und Ermittlung der Keimzahlreduktion
Von jeder Testlösung wurden für jeden Testkeim 20 ml in sterile 50 ml Braunglasflaschen mit Glasperlen abgefüllt und mit 0,2 ml Keimsuspension kontaminiert. Als Kontrollen wurden je Testkeim 20 ml CSL ohne Probe mitgeführt. Die kontaminierten Proben wurden auf der Schüttelmaschine 3 min geschüttelt und bis zu den Entnahmen bei 30 °C im Brutschrank gehalten.
Zu den Entnahmezeitpunkten (1, 2, 3, 24 und 48 Stunden) wurde aus jedem Ansatz 1 ml entnommen, in jeweils 9 ml NaCl-Pepton-Pufferlösung mit EG-Enthemmer überführt und die Koloniezahl ermittelt.
Als 0 Stunden-Werte wurden die Koloniezahlen der verwendeten Testkeimsuspension unter Berücksichtigung der 10⁻²-Verdünnung bei der Probenkontamination angegeben.

### 3. Ergebnisse

Die Einzelergebnisse der Proben sind in folgenden Diagrammen dargestellt. Zusätzlich sind in jedem Diagramm die Keimpopulationen einer wirkstofffreien Blindprobe als Kontrollwert nach 24 Stunden Bebrütung eingetragen.

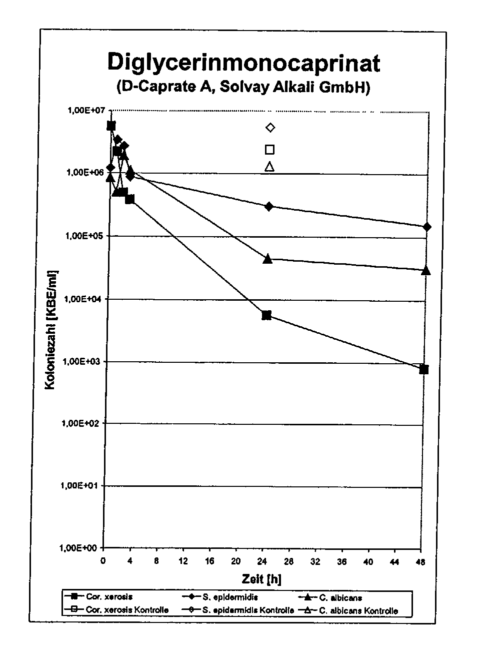

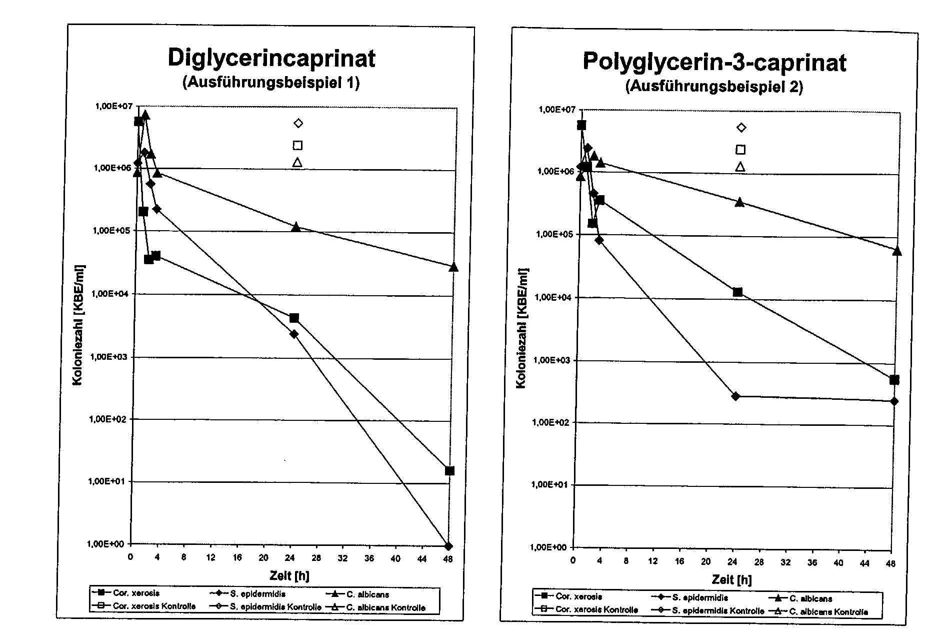

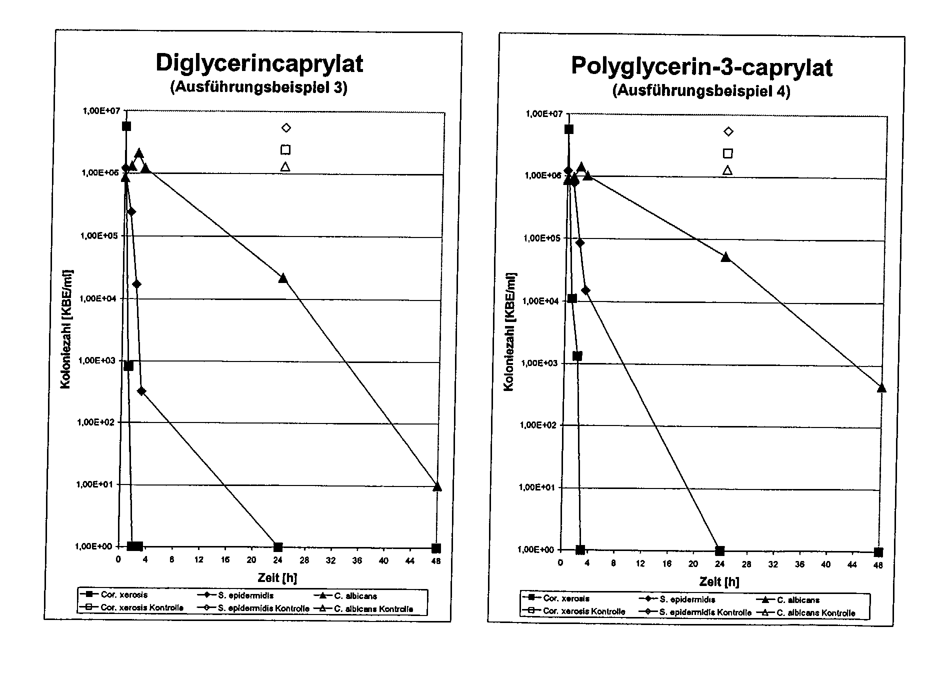

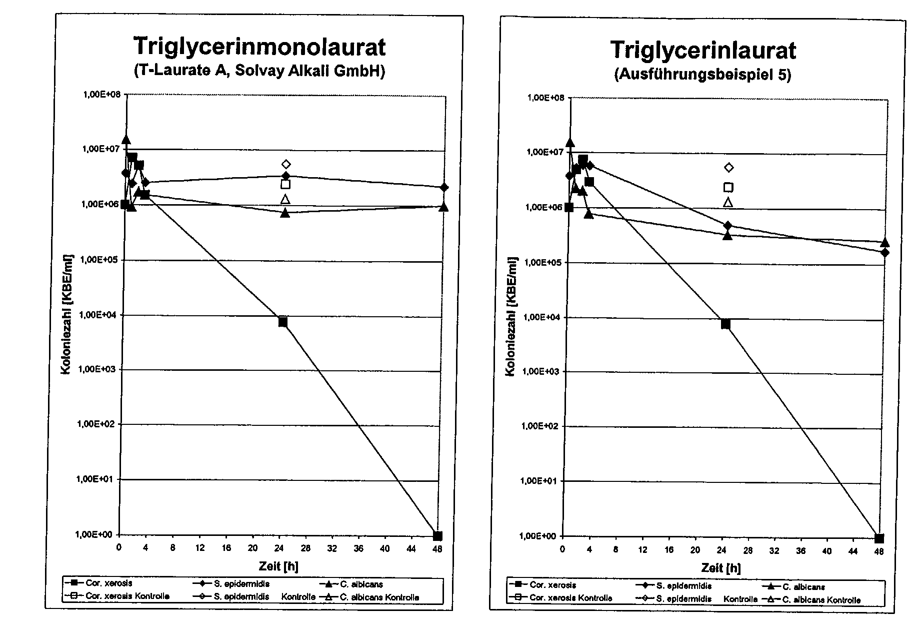

### B) Gegen Malassezia furfur

In gleicher Vorgehensweise wie unter A beschrieben wird die Wirksamkeit von Diglycerincaprylat, wie in Ausführungsbeispiel 3 hergestellt, gegen M. furfur getestet. M. furfur steht in ursächlichem Zusammenhang mit der Bildung von Schuppen.

Diglycerincaprylat wurde in Wasser gelöst, so dass eine Lösung mit 3,0 Gew.-% Gehalt entsteht. Diese Lösung wird mit Keimsuspension versetzt, durch Schütteln homogenisiert und bei 30 °C im Brutschrank gehalten. Eine zweite Lösung ohne Zusatz von Diglycerincaprylat wird als Kontrolle mitgeführt.

Die folgenden Resultate wurden erhalten:

| Probenahme, Zeit (h) | 0 | 1 | 2 | 4 | 24 |
|---|---|---|---|---|---|
| Kontrolle, Keimzahl/ml | 1x10⁵ | n.b. | n.b. | n.b. | 1x10⁴ |
| 0,3 % Diglycerincaprylat, Keimzahl/ml | 1x10⁵ | <10 | <10 | <10 | <10 |
| n.b. = nicht bestimmt | | | | | |

### Beispiel 8: Anwendung in Formulierungen

Es folgen Formulierungen, in denen die erfindungsgemäßen Produkte eingesetzt werden können.

### Formulierung 1: Clear Deodorant Pumpspray

| Phase A: | |
|---|---|
| Produkt aus Beispiel 4 | 0,30 % |
| Trideceth-12 | 2,00 % |
| Dipropylenglykol | 4,00 % |
| Parfüm | 0,90 % |

| Phase B: | |
|---|---|
| Wasser | ad 100,00 |
| Konservierungsmittel | q.s. |
| Zitronensäure (50 %ig) | q.s. |

Die unter Phase A genannten Bestandteile werden unter Rühren in der angegebenen Reihenfolge zusammengefügt und anschließend langsam mit Wasser (Phase B) aufgefüllt. Der pH-Wert wird mit Zitronensäure auf 5,5 eingestellt.

### Formulierung 2: O/W-Emulsion (sprühbar)

| Phase A: | |
|---|---|
| Glycerinstearat (und) Ceteth-20 | 3,00 % |
| (z.B. TEGINACID® H, Degussa) | |
| Stearylalkohol | 1,00 % |
| Produkt aus Beispiel 4 | 0,30 % |
| Dimethicone | 0,50 % |
| Cetearylethylhexanoat | 4,00 % |
| Capryl/Caprin-Triglycerid | 4,00 % |

| Phase B: | |
|---|---|
| Glycerin | 3,00 % |
| Wasser | ad 100,00 % |
| Zitronensäure (50%ig) | pH = 6-7 |
| Konservierungsmittel | q.s. |
| Parfüm | q.s. |

Die Phasen A und B werden auf 70-75 °C erwärmt. Phase A wird unter Rühren zu Phase B gegeben und anschließend homogenisiert. Unter Rühren wird auf 30 °C abgekühlt.
- wichtig:: Falls Phase A vorgelegt werden sollte, muss Phase B ohne Rühren zugegeben werden.

### Formulierung 3: Clear Deodorant Roll On

| Phase A: | |
|---|---|
| Produkt aus Beispiel 4 | 0,30 % |
| Trideceth-12 | 2,00 % |
| Dipropylenglykol | 2,00 % |
| Parfüm | 0,50 % |
| PEG-14 Dimethicone | 1,00 % |
| Wasser | ad 65,00 % |

| Phase B: | |
|---|---|
| Hydroxyethylcellulose (2 % in Wasser) | 35,00 % |
| Konservierungsmittel | q.s. |
| Zitronensäure (50 %ig) | q.s. |

Die unter Phase A genannten Bestandteile werden unter Rühren in der angegebenen Reihenfolge zusammengefügt. Phase A wird unter Rühren zu Phase B gegeben. Der pH-Wert wird mit Zitronensäure auf 5,5 eingestellt.

### Formulierung 4: Anionischer Haushaltsreiniger (Konzentrat)

| Phase A: | |
|---|---|
| Produkt aus Beispiel 4 | 4,00 % |
| Ethanol | 10,00 % |
| Trideceth-12 | 5.00 % |
| Cocamidopropylbetain (∼ 38 % Wirkstoffanteil) | 13,20 % |
| Natriumlaurylethersulfat | 35,80 % |

| Phase B: | |
|---|---|
| Wasser | ad 100,00 % |

Die unter Phase A genannten Bestandteile werden unter Rühren in der angegebenen Reihenfolge zusammengefügt und anschließend langsam mit Wasser (Phase B) aufgefüllt.

### Beispiel 9: Kosmetischer Anwendungstest

Zur Anwendung kommen zwei Formulierungen. Diese sind Formulierung 2 aus Beispiel 6 und als Placebo dieselbe Formulierung, in der das erfindungsgemäße Produkt (aus Beispiel 4) durch nicht verestertes Polyglycerin mit gleicher Verteilung ersetzt worden ist. Der Achselgeruch von 20 Probanden wird vor und nach der Anwendung der Formulierung 2 oder der Placebo-Formulierung durch drei Experten geprüft. Im Einzelnen beinhaltet der Test folgende Schritte:
1. Die Achselhöhle wird mit Seife gewaschen, der Geruch wird durch Experten bewertet,
2. Das Produkt wird einmal in einer Achselhöhle angewendet. Nach 6 und 24 h wird der Geruch geprüft und der Unterschied bewertet.

Das Ergebnis dieser Untersuchung ist, dass sowohl nach 6 als auch nach 24 stündiger Anwendung eine deutliche Verbesserung des Geruches der mit der erfindungsgemäß behandelten Achselhöhle verglichen mit der Placebo behandelten Achselhöhle festgestellt wird.

### Beispiel 10: Konservierung eines Lebensmittels

Kartoffelsalat bestehend aus 750 g gekochten und klein geschnittenen Kartoffeln, 25 g klein geschnittener Zwiebeln, 1,2 g Kochsalz, 10 ml Essig (enthaltend 6 % Essigsäure) und 200 g Mayonaise wird mit 0,5 % des Polyglycerinesters aus Beispiel 4 versetzt. Der Kartoffelsalat wurde zur Überprüfung auf Bakterien und Hefen 72 Stunden bei 30 °C gelagert. Danach wurden die folgenden Keimzahlen bestimmt:

| | |
|---|---|
| Kartoffelsalat ohne Polyglycerinester | 1,2 x 10⁶ Keime/ml |
| Kartoffelsalat mit Polyglycerinester | 1,3 x 10³ Keime/ml |

Zur Überprüfung auf Hefen und Pilze wurde der Kartoffelsalat 72 Stunden bei 25 °C gelagert. Danach wurden die folgenden Keimzahlen bestimmt:

| | |
|---|---|
| Kartoffelsalat ohne Polyglycerinester | 6,7 x 10⁴ Keime/ml |
| Kartoffelsalat mit Polyglycerinester | 2,5 x 10¹ Keime/ml |

Der Kartoffelsalat ohne Polyglycerinester zeigte nach 96 Stunden Lagerung deutlich sichtbaren bläulichen Schimmel, während der Kartoffelsalat mit Polyglycerinester optisch unverändert war.

## Patentansprüche

1. Mittel zur Bekämpfung von Mikroorganismen, **dadurch gekennzeichnet, dass** sie einen Gehalt an Mischungen von Fettsäureestern des Polyglycerins enthaltend primäre und sekundäre Ester des Polyglycerins in einem wirksamen Mol-Verhältnis von 8 : 1 bis 25 : 1 aufweisen.

2. Mittel gemäß Anspruch 1, **dadurch gekennzeichnet, dass** das Verhältnis der primären und sekundären Monoester des Diglycerins einen Wert 10 : 1 bis 20 : 1 aufweist.

3. Mittel gemäß Anspruch 1, **dadurch gekennzeichnet, dass** als Di- und/oder Polyglycerin Verbindungen verwendet werden, wie sie durch Kondensation von Glycerin, durch Hydrolyse und Kondensation von Epichlorhydrin oder durch Polymerisation von Glycidol gewonnen werden.

4. Mittel gemäß Anspruch 1, **dadurch gekennzeichnet, dass** als Säuren und Säurederivate geradkettige oder verzweigte, gegebenenfalls OH-Gruppen und/oder Doppelbindungen enthaltende Fettsäuren mit 6 bis 14 C-Atomen in der Hauptkette verwendet werden.

5. Verwendung von Mitteln gemäß Anspruch 1 zur Bekämpfung von grampositiven Bakterien, gramnegativen Bakterien, Mykobakterien, Dermatophyten, Spross- und Fadenpilzen, Viren und Sporen.

6. Verwendung von Mitteln gemäß Anspruch 1 zur Herstellung von Desinfektionsmitteln, Desinfektionsreinigern, Sterilisationsmitteln, Antiseptika und Konservierungsmitteln.

7. Verwendung von Mitteln gemäß Anspruch 1 zur Konservierung von Lebensmitteln.

8. Verwendung von Mitteln gemäß Anspruch 1 zur antimikrobiellen Ausrüstung von Lebensmittelverpackungen zur Verbesserung der Haltbarkeit des Inhaltes.

9. Verwendung von Mitteln gemäß Anspruch 1 zur Herstellung von kosmetischen Formulierungen für den Bereich der Körperreinigung und Körperpflege.

10. Verwendung von Mitteln gemäß Anspruch 1 zur Herstellung kosmetischer Formulierungen gegen Körpergeruch und gegen Schuppenblildung.

11. Verwendung von Mitteln gemäß Anspruch 1 zur Herstellung von kosmetischen Formulierungen gegen unreine Haut und leichte Formen der Akne.
